# EUROPÄISCHE PATENTSCHRIFT

(11) **EP 0 937 254 B1**
(45) Veröffentlichungstag und Bekanntmachung des Hinweises auf die Patenterteilung: **23.05.2007**
(21) Anmeldenummer: 97948699.0
(22) Anmeldetag: 31.10.1997
(51) Int. Cl.: G01N 33/53

(54) **Monoklonaler Antikörper gegen MxA und MxB**
Monoclonal antibody directed against MxA and MxB
Anticorps dirigé contre les protéines MxA et MxB

(30) Priorität: 31.10.1996 DE 19645010
(43) Veröffentlichungstag der Anmeldung: 25.08.1999
(73) Patentinhaber: Von Wussow, Peter, 30982 Pattensen (DE)
(72) Erfinder: Von Wussow, Peter, 30982 Pattensen (DE)
(74) Vertreter: Pfenning, Meinig & Partner GbR
(86) Internationale Anmeldenummer: PCT/DE1997/002536
(87) Internationale Veröffentlichungsnummer: WO 1998/019160

(56) Entgegenhaltungen:
- EP-A- 0 725 081
- WO-A-95/24500
- OH S-K ET AL: "Quantitation of interferon-induced Mx protein in whole blood lysates by an immunochemiluminescent assay: Elimination of protease activity of cell lysates in toto." JOURNAL OF IMMUNOLOGICAL METHODS 176 (1). 1994. 79-91. ISSN: 0022-1759, XP002059944
- TOWBIN H ET AL: "A WHOLE BLOOD IMMUNOASSAY FOR THE INTERFERON-INDUCIBLE HUMAN MX PROTEIN." J INTERFERON RES 12 (2). 1992. 67-74. CODEN: JIREDJ ISSN: 0197-8357, XP002059945
- HORISBERGER M.A ET AL: "IFN alpha induced human 78kD protein: purification and homologies with the mouse Mx protein, production of monoclonal antibodies and potentiation effect of IFN gamma" J.INTERFERON RES., Bd. 7, Nr. 4, 1987, Seiten 331-343, XP002061585
- WÜTHRICH, RÜDI ET AL: "Monoclonal antibodies detect protein Mx in cells of interferon treated influenza virus resistant cells" BIOL.INTERFERON SYST.PROC. TNO-ISIR (MEETING 1984), 1985, Seiten 317-323, XP002061586
- STAEHELI, PETER ET AL: "Polyclonal and monoclonal antibodies to the interferon-inducible protein Mx of influenza virus-resistant mice" J.BIOL.CHEM., Bd. 260, Nr. 3, 1985, Seiten 1821-1825, XP002061587

## Beschreibung

Die Erfindung betrifft Antikörper zum indirekten Nachweis der Interferon-Produktion mittels Antikörper gegen MxA-Protein und MxB-Protein.

Interferone, insbesondere Interferon α, β und ω, sind eine Klasse natürlich vorkommender Proteine, denen bekanntermaßen eine günstige Wirkung bei viralen Infektionen und bei der Verhinderung von Tumorwachstum zukommt. Weiterhin stimulieren Interferone die Aktivität natürlicher Killerzellen und modulieren die Aktivität von Makrophagen, B- und T-Zellen. Interferone sind somit wichtige Säulen des Immunsystems, allerdings haben zu hohe Interferon-Gehalte auch ungünstige Nebenwirkungen, wie z.B. Knochenmarkstoxizität, ZNS-Toxizität oder Fieber.

Eine Anzahl von Proteinen werden durch Interferon induziert und sind an der antiviralen, antiproliferatorischen und immunmodulatorischen Wirkung, die Interferonen zugeschrieben wird, beteiligt. Zwei dieser Interferon induzierten Proteine sind das MxA- und das MxB-Protein (Markus Aebi et al, Mol. Cell. Biol. 1989, 9: 5062-5072.).

In der WO-A-9524500, sowie in "Journal of Immunological Methods" 176, 1, 1994, 79-91 und in "Journal of Interferon Research" 12, 1992, 67-74, ist die Verwendung von Anti-MxA monoklonalen Antikörper zur in vitro-Messung von Interferon offenbart.

In "Journal of Interferon Research" 7, 1987, 331-343 sind monoklonale Antikörper die gegen ein 78 kD-Protein gerichtet sind, bekannt. Hierbei handelt es sich um ein humanes MxA-Protein. Weiter ist in "Biol. Interferon Syst. Proc." TNO-ISIR (Meeting 1984), 1985, 317-323 ein Antikörper offenbart, der gegen ein aus Mauszellen gewonnenes Mx-Protein gerichtet ist.

In der EP-A-0 725 081 ist offenbart, dass Mx-Protein durch Interferon induziert wird. Eine Möglichkeit wie eine festgestellte zu hohe bzw. zu niedrige Interferon-Produktion korrigiert werden kann, ist hieraus nicht zu entnehmen.

Da man mittels der Bestimmung der endogenen Interferonproduktion eine Aussage über die Aktivität des immunsystems machen kann und man bei verschiedenen Erkrankungen wissen muß, ob eine zu hohe oder zu niedrige Interferonproduktion stattfindet, besteht die Aufgabe der vorliegenden Erfindung darin, Antikörper zur Verfügung zu stellen mit denen die Interferonproduktion einfach bestimmt werden kann.

Gelöst wird diese Aufgabe durch die Antikörper nach Anspruch 1.

Von den Erfindern wurde gefunden, daß die Typ I Interferonproduktion in vivo unter Verwendung von Anti-körpern gegen , MxA- und MxB-Protein, bestimmt werden kann. Zwischen der Produktion von Interferonen in einem Patienten und der Expression der Interferon-induzierten Proteine MxA und/oder MxB besteht eine Korrelation, d.h. bei einer zu hohen Interferonproduktion im Körper ist auch die Konzentration an MxA und MxB erhöht bzw. bei einer zu geringen Produktion an Interferon erniedrigt.

Erfindungsgemäß erfolgt die Bestimmung des MxA und MxB-Gehalts immunologisch durch Verwendung von Antikörpern gegen diese Proteine. Erfindungsgemäß ist es möglich, polyklonale oder monoklonale Antikörper bzw. dessen Fragmente oder Konjugate zu verwenden.

Zur Herstellung der Antikörper wird zuerst Mx1- und/oder MxB-Protein gewonnen. Dies geschieht vorzugsweise dadurch, daß murine oder menschliche Zellen, z.B. Lymphozyten, Makrophagen, Monozyten oder lymphoblastoide Zellen in einem gewöhnlichen Wachstumsmedium (z.B. RPMI 1640 Medium) ggf. angereichert mit Vitaminen und/oder Hormonen kultiviert werden. Nach Ende der exponentiellen Wachstumsphase werden die Zellen mit natürlichem oder rekombinantem Interferon inkubiert, und zwar in Konzentrationen von 5 x 10⁴ bis 10⁸ Zellen/ml und 3.000 bis 12.000 internationalen Interferoneinheiten/ml. Die Zellen werden nachfolgend geerntet und gemäß Standardmethoden lysiert. Das bzw. die Proteine werden aufgereinigt, z.B. mittels Säulenchromatographie, HPLC oder Elektrophorese und als Antigene zur Erzeugung von Antikörpern verwendet. Auch eine rekombinante Herstellung ist möglich.

Polyklonale Antikörper werden mittels Standardmethoden dadurch erhalten, daß das aufgereinigte Mx1- und/oder MxB-Protein Mäusen oder Kaninchen injiziert wird und aus dem dem immunisierten Tier entnommenen Blutserum, z.B. mittels Affinitätschromatographie, Antikörper erhalten werden.

Monoklonale Antikörper werden mittels Standardmethoden dadurch erhalten, daß vorzugsweise einer Mx-negativen Maus aufgereingtes Mx1- und/oder MxB-Protein injiziert wird, Antikörper-produzierende Zellen (z.B. die Milz) des immunisierten Tiers entnommen werden und mit Myelomazellen fusioniert werden, wobei die fusionierten Zellen anschließend geklont werden. Die dabei erhaltenen Hydridomzellen werden in vitro kultiviert (z.B in Dulbecco's Modifiziertem Eagle Medium oder RPMI 1640 Medium) und die monoklonalen Antikörper aus dem Kulturüberstand gewonnen. Dafür eignen sich dem Fachmann bekannte Verfahren, wie Ammoniumsulfatfällung und nachfolgende Aufreinigung der Immunglobuline durch Standardchromatographieverfahren.

Von den Erfindern wurden monoklonale Antikörper gegen MxB Protein bei der DSM, Braunschweig, am 16.06.1997 unter der Nummer DSM-ACC 2308 (interne Bezeichnung 8-271) und der Nummer DSM-ACC 2309 (interne Nummer 7-88) hinterlegt. Monoklonale Antikörper gegen MxA + MxB Protein wurden unter der Nummer DSM-ACC 2289 (interne Nummer 2-95) und DSM-ACC 2290 (interne Nummer 5-237) am 06.12.1996 hinterlegt.

Fragmente der Antikörper, z.B. Fab, Fab' oder F(ab')₂-Fragmente können erhalten werden, indem die vorstehend erhaltenen Antikörper mit Enzymen (z.B. Pepsin oder Papain) verdaut werden oder die Disulfidbindungen chemisch reduziert werden.

Es können auch Konjugate der Antikörper oder von dessen Fragmenten hergestellt werden, z.B. indem die Antikörper oder deren Fragmente mit Glutaraldehyd, Biotin oder Avidin gekoppelt werden.

Erfindungsgemäß soll der Begriff Antikörper so verstanden werden, daß es sich entweder um monoklonale oder polyklonale Antikörper sowie deren Fragmente oder Konjugate handelt.

Von den Erfindern wurden folgende neue Zusammenhänge aufgeklärt:
1) Die Mx-Proteine zeigen Krankheitsaktivität beim systemischen Lupus erythematodes (SLE) und der Mischkollagenose an;
2) Die Mx-Proteine zeigen den Progress der HIV-Infektion an und sind damit ein prognostischer Marker für das Überleben der Patienten;
3) Beim Färben von histologischen Schnitten zeigen Mx-Antikörper SLE-Aktivität in Nierenschnitten an;
4) Beim Färben von histologischen Schnitten von Transplantatgewebe zeigen die Mx-Antikörper eine Abstoßung an.
5) Bei in vitro Kulturen mit menschlichem HIV-Infizierten, mononukleären Zellen werden hohe Mx-Spiegel gemessen. In Gegenwart von antiretroviral wirksamen Medikamenten sinken diese Mx-Spiegel.

Allen Anwendungen gemeinsam ist, daß Killerzellen, insbesondere T-Zellen vermittelte Attacken auf humane Zellen diese Zellen dazu anregen, Mx-Proteine zu exprimieren.

Von den Erfindern wurde weiter gefunden, daß AIDS-Patienten stadienabhängig vermehrt Interferon produzieren. Diese Patienten produzieren z.T. mehr Interferon als Patienten, die Interferon-α als Medikament bekommen. Patienten, die therapeutisch Interferon-α erhalten, haben meistens unter Nebenwirkungen, wie Knochenmarkstoxizität, ZNS-Toxizität oder Fieber, zu leiden. Genau diese Symptome haben auch AIDS-Patienten, die zuviel Interferon produzieren.

Ähnlich verhält es sich bei Patienten, die an einer Autoimmunkrankheit leiden oder eine Steroidtherapie erhalten. Dort genügt schon eine kurzfristige Gabe von Steroiden, z.B. von Kortisonen (z.B. Prednisolon), um den Mx-Gehalt unter einen bestimmten Spiegel sinken zu lassen.

Von den Erfindern wurde nun gefunden, daß das Steigen oder Sinken der endogenen Interferonproduktion (Interferon Typ I) leicht durch Nachweis von erhöhten oder erniedrigten MxA- und MxB-Proteinspiegeln in zellhaltigen Körperflüssigkeiten (z.B. Blut, Urin, Liquor) nachgewiesen werden kann. Dies geschieht dadurch, daß in geeigneten immunologischen Nachweisverfahren, z.B. ELISA oder RIA, unter Verwendung von Antikörpern oder dessen Fragmenten oder Konjugaten gegen MxA- und MxB-Protein der Gehalt an MxA- und MxB-Protein nachgewiesen wird. Aus einem erhöhten bzw. erniedrigten Gehalt an MxA-Protein und MxB-Protein in der untersuchten Körperflüssigkeit des Patienten kann dann auf eine erhöhte bzw. erniedrigte Interferonproduktion im Patienten geschlossen werden. Es wurde herausgefunden, daß eine Mx-Konzentration von 0,5 bis 2,0 mU/1000 Leukozyten als normal gilt und eine normale Interferonproduktion im Patienten anzeigt.

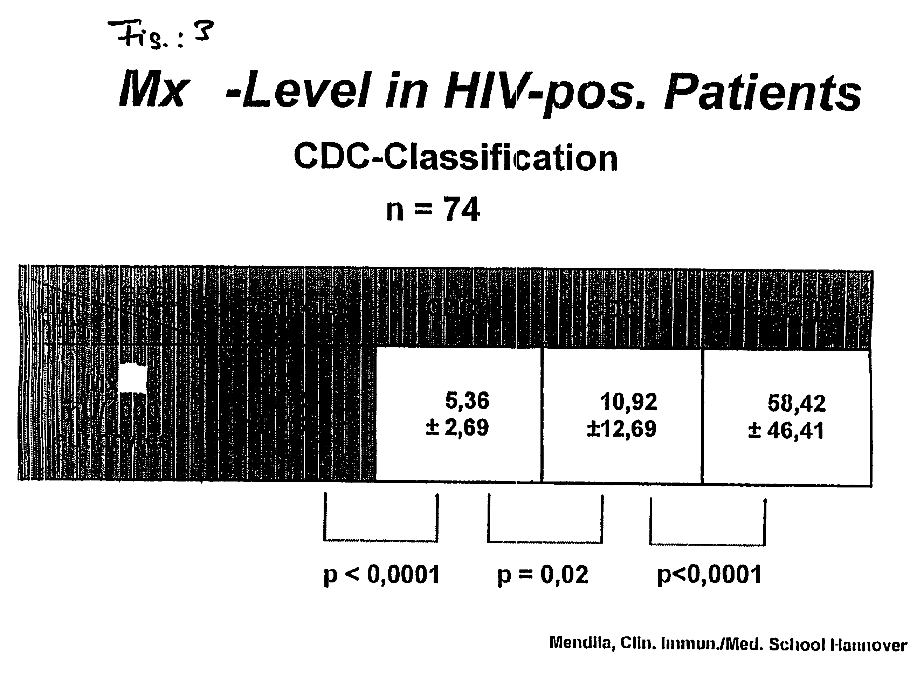

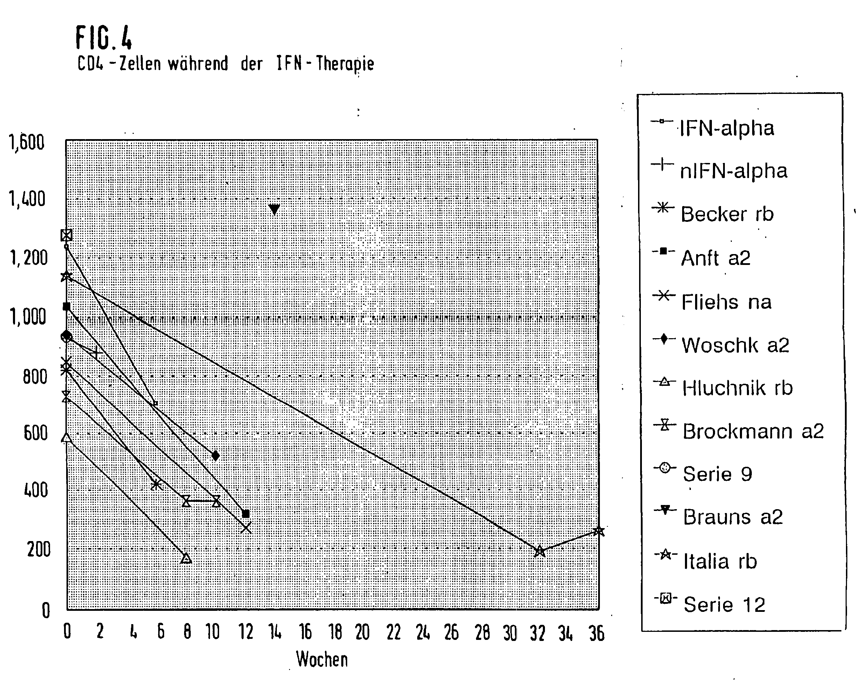

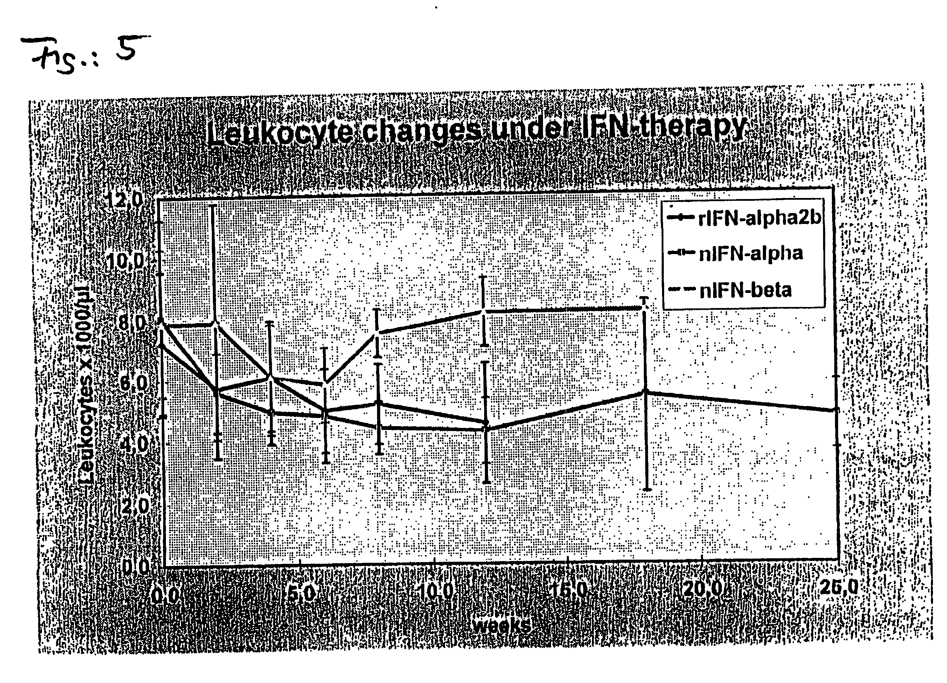

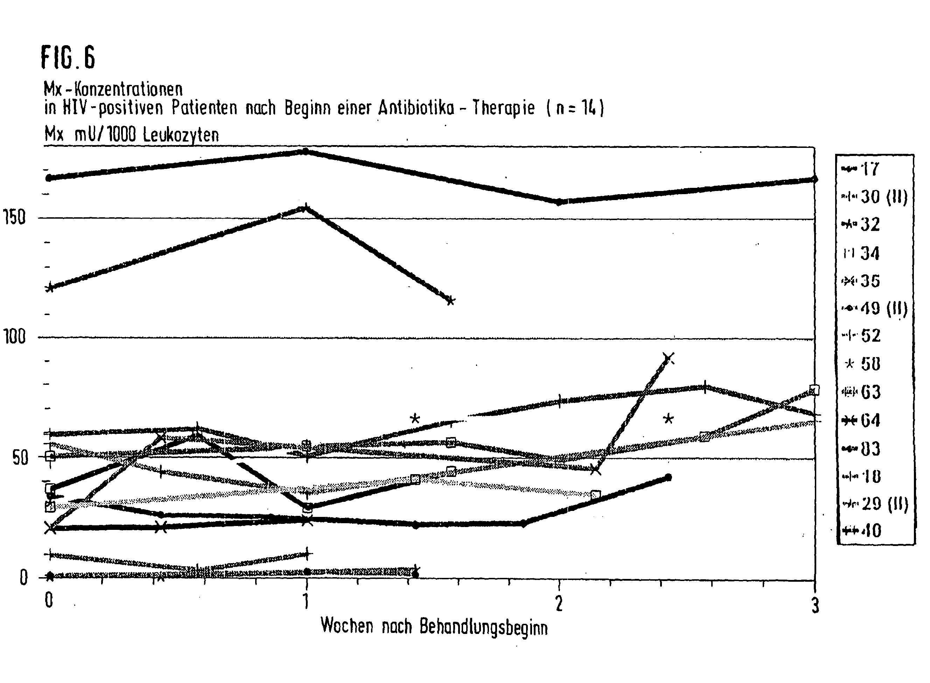

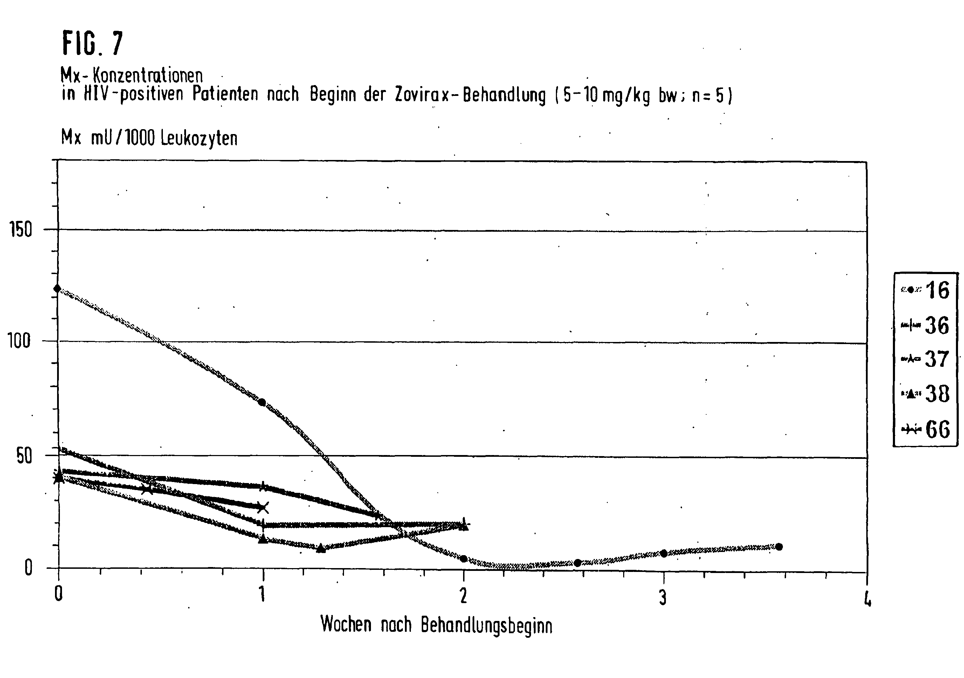

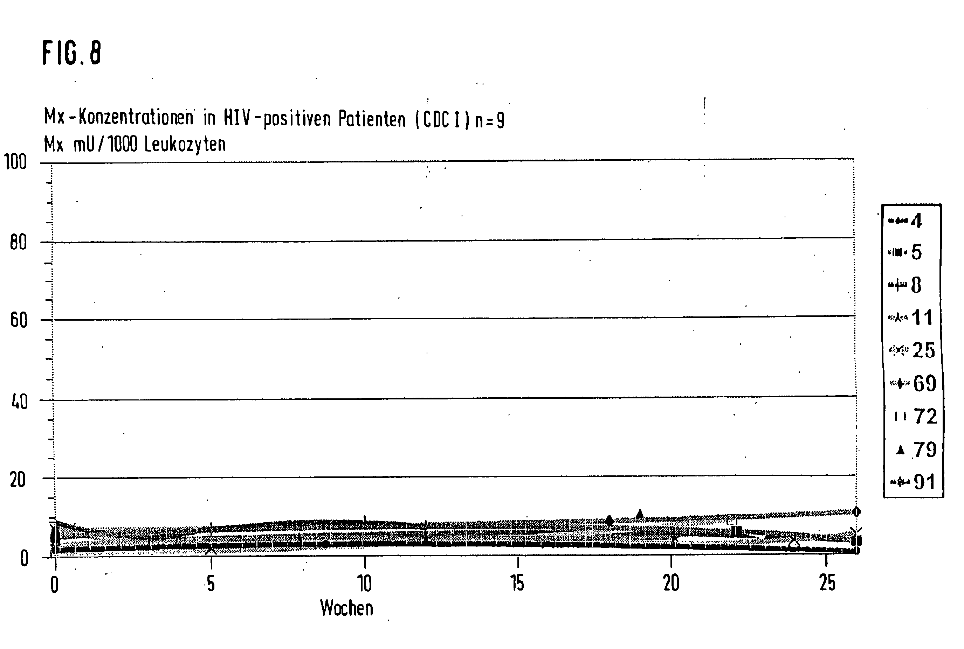

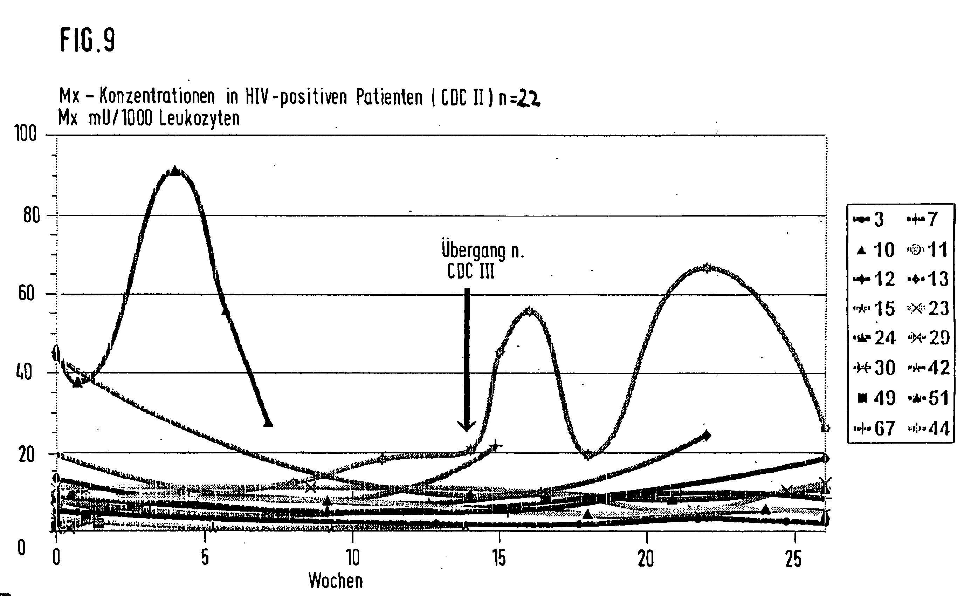

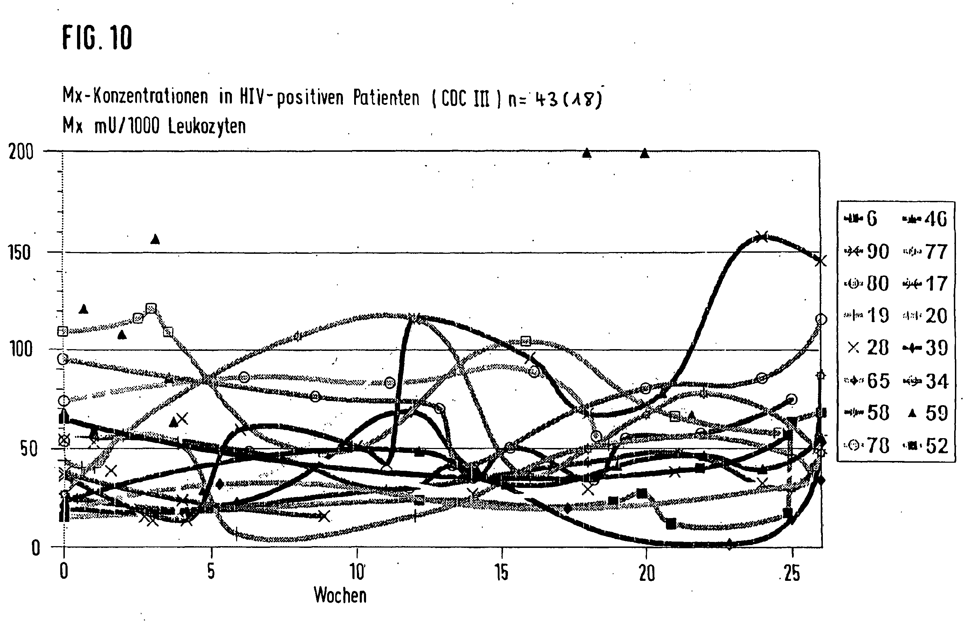

So wird gemäß vorliegender Erfindung beim Auffinden eines erhöhten MxA- und MxB-Wertes Anti-Interferon-Mittel (z.B. Steroide, Antiinterferonantikörper) in einer geeigneten Mengen verabreicht, daß die oben erwähnten Normwerte an MxA und MxB wieder erreicht werden. Eine solche Behandlung hat sich als sehr vorteilhaft erwiesen, um die bei AIDS auftretenden Begleiterscheinungen zu vermeiden oder zu lindern. Die gemäß Verfahren durchgeführte Mx-Messung erlaubt die Bestimmung der Typ I Interferonproduktion in Patienten mit einer HIV-Infektion. Überraschenderweise hat sich bei den Untersuchungen der Erfinder gezeigt, daß alle unbehandelte HIV-infizierte Patienten unabhängig vom Stadium einen erhöhten Mx-Spiegel besitzen und daß die Mx-Spiegel mit Voranschreiten der Erkrankung signifikant zunehmen. In vitro mit antiretroviral wirksamen Substanzen inkubierte Blutmononukleäre Zellen von HIV-Infizierten exprimieren wesentlich weniger Mx.Daher können so antiviral wirksame Substanzen indentifiziert werden. Es ist bekannt, daß exogen verabreichtes IFN-α, wenn es längerfristig und insbesondere in hohen Dosen gegeben wird, im Menschen eine Leukopenie und polyneuropathische/depressive Sympotome sowie erniedrigde CD4-Zellzahlen verursacht. Genau diese Symptome finden sich auch, wenn bei HIV-infizierten Patienten hohe Mx-Konzentrationen auftreten. Überraschenderweise korrelieren Leukopenie und polyneuropathische/depressive Symptome bei AIDS-Patienten mit der Höhe der Mx-Proteine und damit mit der Höhe der endogen gebildeten IFN-Menge. Man muß also dafür sorgen, daß weniger aktives Interferon im AIDS-Patienten vorkommt. So wird beispielsweise eine Anti-IFN-α-Antikörper-Therapie oder Anti-IFN-α,β-Rezeptor-Antikörper-Therapie durchgeführt, die das endogene IFN blockiert und reduziert bzw. behebt dadurch die Leukopenie und die polyneuropathischen/depressiven Beschwerden. Diese Therapie muß längerfristig (ca. 4 Wochen bis 6 Monate) durchgeführt werden. In diesem Zusammenhang wird auf die Figuren 3 bis 10 hingewiesen.

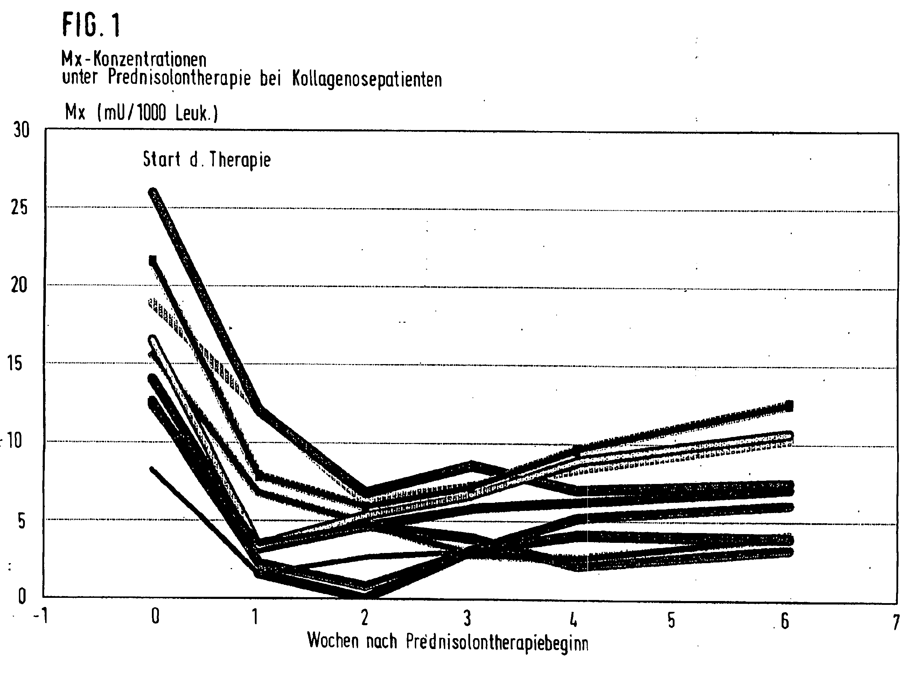

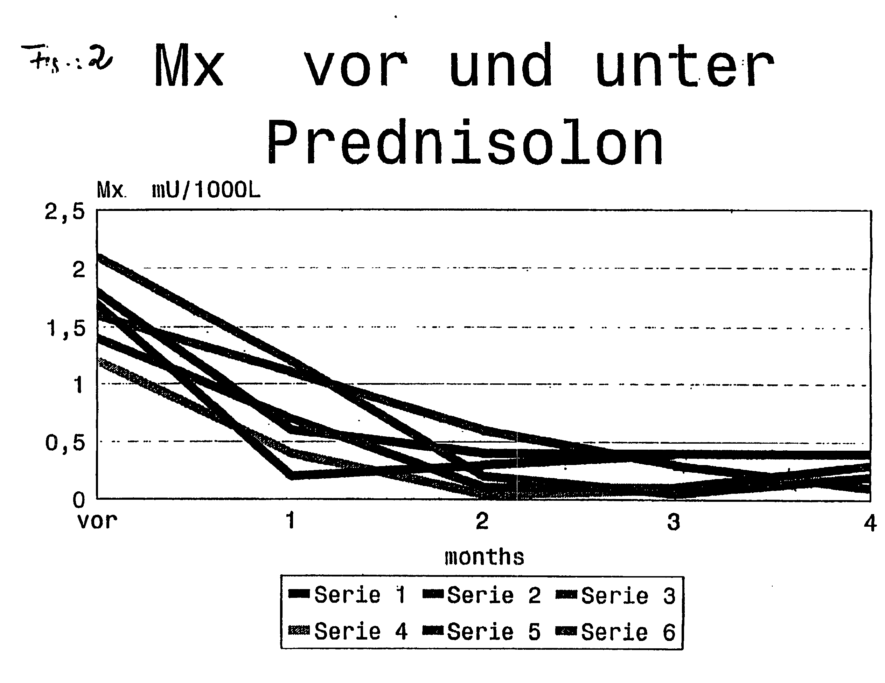

Umgekehrt kann beim Auffinden zu geringer Mx-Konzentrationen Interferon (Typ I) verabreicht werden, um die oben genannten Normwerte wieder zu erreichen. Dadurch werden z.B. einige der bekannten Nebenwirkungen bei der Steroid-Therapie vermieden bzw. entscheidend gelindert. So führt eine Steroidtherapie mit Dosen über 20 mg Prednisolon täglich zu einer Infektanfälligkeit, vor allem gegenüber Viren, da die endogene IFN-Produktion erniedrigt wird und so der Mx-Spiegel sinkt. Auch Steroide in mittelhohen Dosen reduzieren den Mx-Spiegel von 2 mU auf 0,2 mU pro 1000 Leukozyten. Da erhöhte Mx-Werte mit einem Schutz gegenüber Viren assoziiert sind, ist zu erwarten, daß eine Erniedrigung der endogenen IFN-Produktion mit einer erhöhten Anfälligkeit gegen Viren verbunden ist. Um dieser Entwicklung entgegenzuwirken, empfiehlt sich eine niedrig-dosierte IFN-Therapie, da dadurch auch in Gegenwart von Steroiden die Mx-Konzentration im peripheren Blut erhöht wird. Von den Erfindern wurde ermittelt, daß eine niedrig-dosierte IFN-Therapie (z.B. 200.000 bis 1.000.000 U/Tag IFN-α) die Infektanfälligkeit, die durch Steroidgaben hervorgerufen worden ist, beheben kann. In diesem Zusammenhang wird auf die Figuren 1 und 2 hingewiesen.

Von den Erfindern wurden außerdem Mx-Messungen zur Aktivitätsüberwachung von Patienten mit Autoimmunerkrankungen und Transplantationen durchgeführt. Patienten mit systemischem Lupus erythomatodes (SLE) und solche mit Kollagenosen (MCTD) sind überwiegend Mx-positiv, sofern sie eine Krankheitsaktivität aufweisen und keine Steroide medikamentös erhalten.
Auch Patienten mit subcutanem Lupus erythomatodes sind im Blut Mx-positiv. Patienten mit discoidem Lupus haben im Blut normale Mx-Konzentrationen, aber erhöhte Mx-Werte in der Haut.

Patienten mit SLE und solche mit MCTD haben im inaktiven Stadium ihrer Erkrankung normale Mx-Werte. Patienten im aktiven Stadium haben erhöhte Mx-Werte und somit auch eine erhöhte endogene Interferon-Produktion. Unter der Therapie mit Steroiden fallen die Mx-Werte dann wieder, so daß sogar noch Interferongaben angezeigt sein können. Von den Erfindern wurde jedenfalls eine enge Korrelation der Mx-Werte von peripherem Blut und Krankheitsaktivität festgestellt.

Mx-Färbungen zeigen, daß Granulozyten, Lymphozyten und Monozyten sowie ein Teil der Placenta MxA-positiv werden können. Normale Haut ist Mx-negativ, Patienten mit einer Typ I Interferontherapie haben intensiv Mxpositive Keratinozyten, Muskelzellen, Schweiß- und Talkdrüsenzellen. Außerdem wurde festgestellt, daß Transplantationsgewebe (Herz) während der Abstoßungsreaktion Mx-positiv ist und somit eine erhöhtee Interferonproduktion vorliegt, die mittels geeigneter Anti-Interferon-Mittel gesenkt werden kann.

Die Erfindung wird nun weiter mit Bezug auf die Figuren beschrieben, die zeigen:
- Fig. 1:: Mx-Level unter Prednisolontherapie bei Kollagenosepatienten
- Fig. 2:: Mx-Level vor und nach der Therapie mit Prednisolon bei Tumorpatienten
- Fig. 3:: Mx-Level in HIV-positiven Patienten
- Fig. 4:: CD4-Zellen während der IFN-Therapie
- Fig. 5:: Leukozytenveränderungen unter der IFN-Therapie
- Fig. 6:: Mx-Level in HIV-positiven Patienten nach Beginn einer Antibiotika-Therapie
- Fig. 7:: Mx-Level in HIV-positiven Patienten nach Beginn der Zovirax-Behandlung
- Fig. 8:: Mx-Level in HIV-positiven Patienten (CDC I)
- Fig. 9:: Mx-Level in HIV-positiven Patienten (CDC II)
- Fig. 10:: Mx-Level in HIV-positiven Patienten (CDC III)

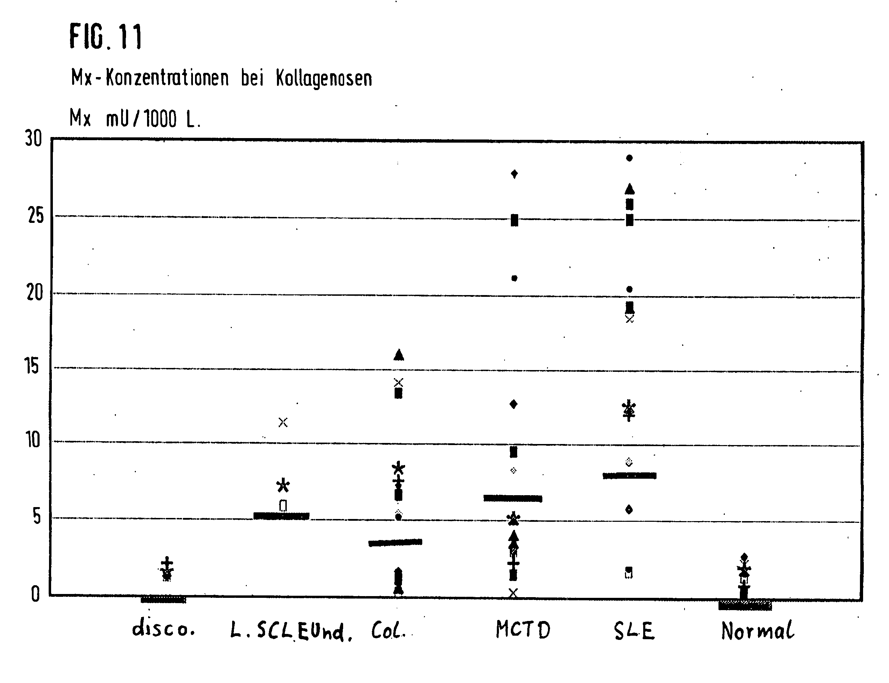

- Fig. 11:: Mx-Level bei Kollagenosen

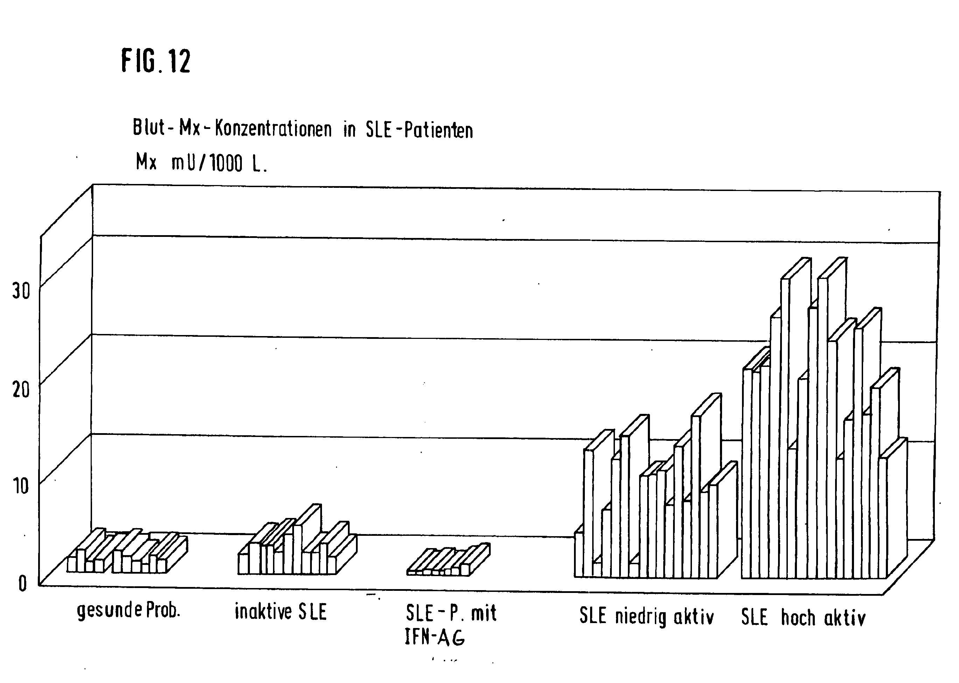

- Fig. 12:: Blut-MxA-Level in SLE-Patienten

Weiter wird die Erfindung gemäß nachfolgender Beispiele beschrieben:

### BEISPIEL 1

### Gewinnung eines monoklonalen Antikörpers, der, gegen MxA und/oder MxB gerichtet ist

Weibliche Balb/c Mäuse werden mit 10 µg rekombinanten MxB subcutan injiziert. Am Tag 27 erhalten die Mäuse rekombinantes Mx1, ebenfalls 10 µg. Am Tag 51 erhalten die Mäuse erneut 10 µg MxB intravenös. Am Tag 55 werden die Mäuse getötet, deren Milz entnommen und diese Milzzellen mit der SP2-O Myelomzellinie fusioniert (Standardverfahren). Überstände der Hybridomkulturen werden in einem Screening-Verfahren getestet. Geeignete positive Kulturen werden einem Limiting-dilution-Verfahren unterzogen und so Hybridom-Klone gewonnen.

Die Klone 2308 und 2309 / 2289 und 2290 werden in der Kultur expandiert und in eine Technomouse gesetzt. Die so gewonnenen antikörperhaltigen Überstände werden mittels Protein A-Chromatographie aufgereinigt.

In 96 Loch-Platten werden entweder rekombinantes Mx1, MxA oder MxB in einer Konzentration von 5 *µ*g/ml für 16 Std. bei 4°C gecoated. Nachfolgend erfolgt eine Blockierung mit Rinderalbumin. Nach Waschen der Platten werden die Hybridomüberstände für 24 Std. bei Raumtemperatur in dieser Platte inkubiert. Nach erneutem Waschen werden Alkalische-Phosphate-gekoppelte Anti-Maus-IgG-Antikörper vom Schaf für 1 Std. zu der Platte gegeben und erneut gewaschen. Zur Entwicklung der Farbreaktion wird p-Nitrophenylphosphatase verwendet. Es werden diejenigen Klone selektiert, die sowohl MxA als auch MxB oder nur MxB erkennen.

### BEISPIEL 2

### MxA und MxB-spezifischer ELISA

Ein monoklonaler Antikörper wird nach gängigem Verfahren biotinyliert. Nachfolgend wird ein ELISA mit diesen beiden Antikörpern aufgebaut.

20 µl von lysiertem Citratblut oder einer Zellsuspension werden für 16 Std. bei Raumtemperatur auf 96 Lochplatten gegeben, die mit einem nicht biotinylierten monoklonalen Antikörper (5 µg/ml) vorgecoated waren. Nach ausführlichem Waschen wird der biotinylierte Antikörnper für 2 Std. hinzugegeben. Nach erneutem Waschen wird ein Avidin-alkalische Phosphatase-Konjugat hinzugegeben. Nach einem weiteren Waschschritt wird die Intensität einer Farbreaktion durch Hinzugabe von P-Nitrophenylen-Phosphat bei 405 nm gemessen. Dieser ELISA erfaßt, je nach eingesetztem Antikörper, MxA und MxB oder nur MxB annähernd gleich gut. Die Mx-Konzentration im Blut wird in mU/1000 Leukozyten angegeben.

## Patentansprüche

1. Monoklonaler Antikörper, der gegen MxA und MxB gerichtet ist.

2. Monoklonale Antikörper nach Anspruch 1 mit der Hinterlegungsnummer DSM-ACC 2289, interne Nummer 2-95, sowie DSM-ACC 2290 interne Nummer 5-237.

## Claims

1. Monoclonal antibody directed against MxA and MxB.

2. Monoclonal antibodies according to Claim 1 with the deposition number DSM-ACC 2289, internal number 2-95, and DSM-ACC 2290, internal number 5-237.

## Revendications

1. Anticorps monoclonal dirigé contre MxA and MxB.

2. Anticorps monoclonaux selon la revendication 1 ayant le numéro d'ordre du dépôt DSM-ACC 2289, le numéro interne 2-95, et DSM-ACC 2290, le numéro interne 5-237.
